(19) Europäisches Patentamt
European Patent Office
Office européen des brevets

(11) EP 1 759 634 B1

(12) EUROPÄISCHE PATENTSCHRIFT

(45) Veröffentlichungstag und Bekanntmachung des Hinweises auf die Patenterteilung:
**05.05.2010 Patentblatt 2010/18**

(51) Int Cl.:
*A61B 5/08* *(2006.01)*    *A61M 16/00* *(2006.01)*
*A61K 49/00* *(2006.01)*

(21) Anmeldenummer: **06018250.8**

(22) Anmeldetag: **31.08.2006**

(54) **Verwendung von 1,1,1,2-Tetrafluorethan zur Lungenfunktionsmessung**

Use of 1,1,1,2-Tetrafluoroethane for lung function evaluation

Utilisation de 1,1,1,2-Tetrafluoroethane pour évaluer la fonction pulmonaire

(84) Benannte Vertragsstaaten:
**DE FR GB**

(30) Priorität: **03.09.2005 DE 102005041905**

(43) Veröffentlichungstag der Anmeldung:
**07.03.2007 Patentblatt 2007/10**

(73) Patentinhaber: **Dräger Medical AG & Co. KG**
**23542 Lübeck (DE)**

(72) Erfinder:
• **Dicks, Bernd-Michael**
**23564 Lübeck (DE)**
• **Koch, Joachim**
**23909 Ratzeburg (DE)**

(74) Vertreter: **UEXKÜLL & STOLBERG**
**Patentanwälte**
**Beselerstraße 4**
**22607 Hamburg (DE)**

(56) Entgegenhaltungen:
**DE-A1- 10 046 465    US-B1- 6 254 546**

• **PIKE VICTOR W; AIGBIRHIO FRANKLIN I; FREEMANTLE CHRISTOPHER A J;PAGE BRIAN C;RHODES CHRISTOPHER G; WATERS S L; JONES T; ET AL: "Disposition of inhaled 1,1,1,2-tetrafluoroethane (HFA134A) in healthy subjects and in patients with chronic airflow limitation: Measurement by 18F-Labeling and Whole-Body gamma-Counting" DRUG METABOLISM AND DISPOSITION, Bd. 23, Nr. 8, 1995, Seiten 832-839, XP008104470**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 759 634 B1

**Beschreibung**

Die Erfindung betrifft die Verwendung von 1,1,1,2-Tetrafluorethan zur Lungenfunktionsmessung, speziell zur Bestimmung der funktionellen Residualkapazität (FRC) von Lungen während einer Beatmung.

Die Lungenfunktionsmessung und speziell die FRC-Bestimmung bei der Beatmung bei Verwendung von Fluorpropan, nämlich Heptafluorpropan, Hexafluorpropan oder Perfluorpropan als Spuren-/Tracergase geht aus DE 100 46 465 B4 und der äquivalenten US 6,544,191 B2 hervor, so dass für das Verfahren zur Lungenfunktionsmessung und FRC-Bestimmung auf diese Vorveröffentlichungen explizit Bezug genommen wird.

In der US 6,254,546 B1 wird ein Verfahren zur Bestimmung von Lungeneigenschaften außerhalb der maschinellen Beatmung mit Hilfe einer Bolusdosierung eines Gemisches unterschiedlich blutlöslicher Gase beschrieben, wobei die Messauswertung mit Hilfe eines Optimierungsalgorithmus für die im Anschluss an die Inhalation gemessenen exspiratorischen Gaskonzentrationen erfolgt.

Darüber hinaus sind verschiedene weitere Spurengase für die FRC-Messung verwendet worden mit Zugaben zum Atemgas von im Allgemeinen wenigen Vol. %, wodurch die gesundheitliche Belastung des beatmeten und untersuchten Patienten weitestgehend reduziert und Risiken vermieden werden.

Nachteil der bisher verwendeten Spuren- / Tracergase sind hohe Beschaffungskosten und der im Allgemeinen große technische Aufwand, mit dem die Abfüllung und Applikation der verwendeten Spurengase erfolgt mit der Folge, dass sich die Lungenfunktionsdiagnostik mittels Spurengasen bisher nur in Nischenmärkten wie der Hochleistungssportmedizin etabliert hat und insbesondere nicht zur klinischen Routine bei maschinell beatmeten Patienten auf Intensivstationen gehört. Einige Spurengase wie zum Beispiel Helium erfordern zudem einen hohen messtechnischen Aufwand durch die anzuwendende massenspektrometrische Bestimmung, andere wie beispielsweise Schwefelhexafluorid verfügen in den meisten Ländern nicht über eine medizinische Zulassung für die medizinische Verwendung.

Somit besteht die Aufgabe der Erfindung in der Auswahl eines Spurengases zur Verwendung für die Lungenfunktionsmessung und speziell die FRC-Bestimmung der Lunge eines Patienten oder Probanden bei der Beatmung, das einfach nachweisbar, physiologisch unbedenklich, umweltverträglich und kostengünstig verfügbar ist.

Die Lösung der Aufgabe wird gemäß Anspruch 1 erreicht durch die Verwendung des praktisch weltweit verbreiteten, medizinisch zugelassenen, aber für die FRC-Messung bisher nicht verwendeten Fluorkohlenwasserstoffs 1,1,1,2-Tetrafluorethan mit der Formel $F_3C-CH_2F$ und entsprechender Summenformel $C_2H_2F_4$, INN-Bezeichnung Norfluran; weitere Bezeichnung: "R 134a". Wesentliche Vorteile des 1,1,1,2-Tetrafluorethans, auch im Vergleich zum ebenfalls medizinisch zugelassenen 1,1,1,2,3,3,3-Heptafluorpropan (weitere Bezeichnung: "R 227") sind:

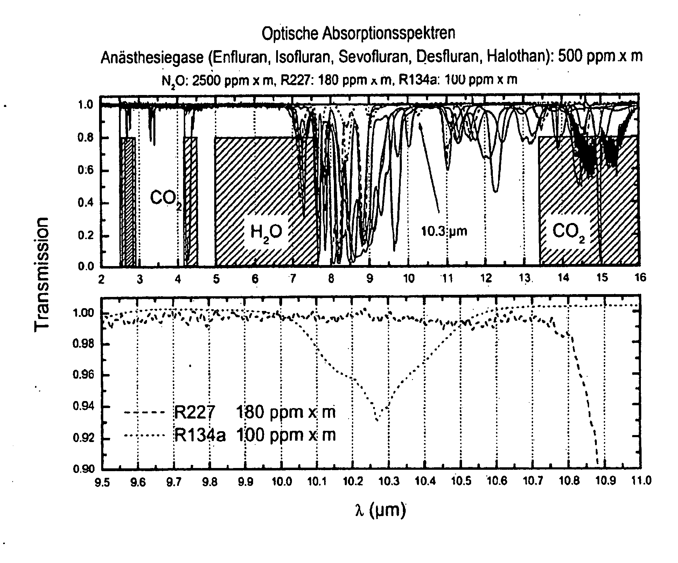

- Eine optische Absorptionslinie bei 10,3 Mikrometern, wo die Querempfindlichkeit auf bei der maschinellen Beatmung unter Anästhesie vorkommende Anästhetika und Lachgas ($N_2O$) weitgehend reduziert ist, wie in der einzigen Figur zu erkennen ist, wo im oberen Teil die Absorptionsspektren der unterschiedlichen bekannten Anästhesiegase (Enfluran, Isofluran, Sevofluran, Desfluran, Halothan) und Lachgas ($N_2O$) über der Wellenlänge $\lambda$ aufgetragen sind sowie im unteren Teil die Absorptionsspektren der Fluorkohlenwasserstoffe 1,1,1,2-Tetrafluorethan ("R 134a") sowie 1,1,1,2,3,3,3-Heptafluorpropan ("R 227"). Aus der Figur ist ersichtlich, dass bei Verwendung von "R 134a" auch bei Anwesenheit von volatilen Anästhetika und Lachgas ($N_2O$) Infrarot-Konzentrationsmessungen ohne Weiteres aufgrund des charakteristischen Absorptionspeaks bei 10,3 Mikrometern möglich sind, während dies für "R 227" nicht möglich ist.

Weitere Vorteile sind der geringere Einkaufspreis von "R 134a" im Vergleich zu "R 227" aufgrund des einfacheren Herstellungsprozesses und der stärkeren Verbreitung sowie die bessere Umweltverträglichkeit mit einem geringeren "Global Warming Potential" (GWP) bezogen auf $CO_2$ von 1300 ("R 227": 2900).

Die Applikation des Tracergases kann entweder direkt aus einem Inhalator in Form einer Bolusdosierung bei der Beatmung erfolgen oder mit Hilfe eines zwischengeschalteten Dosierers, der kontinuierlich oder stoßweise betrieben wird und dabei optional das Tracergas mit anderen Atemgasen im Anästhesie- oder Beatmungsgerät mischt. Als Inhalat dient dabei beispielsweise ein Arzneimittel, in dessen Zusammensetzung "R 134a" als Zusatzstoff einer fertigen Inhalationslösung enthalten ist oder eine Teilmenge der Zusammensetzung, die ebenfalls "R 134a" enthält.

Die FRC-Bestimmung mit "R 134a" als Spuren- / Tracergas erfolgt, wie aus dem Stand der Technik bekannt, speziell durch eine Kombination aus Volumen- und Konzentrationsmessung mit entsprechender Auswertung, wie in DE 100 46 465 B4 und US 6,544,191 B2 angegeben.

Die FRC-Bestimmung erfolgt an maschinell beatmeten Patienten. Durch Messung weiterer Bestandteile der Inhalationszusammensetzung sind optional weitere, an sich bekannte funktionale Größen wie "Pulmonary Blood Flow" berechenbar, auch kann eine an sich bekannte Kompartmentanalyse der Lunge wie folgt durchgeführt werden:

Trägt man nach einem Tracergasauswasch die endexspiratorischen Konzentrationen semilogarithmisch über der Zahl der Atemzüge seit Beginn des Auswaschs auf, so erhält man im Allgemeinen einen multilinearen Verlauf. Jede lineare Komponente entspricht einer Auswaschzeitkonstanten und kann einem Lungenkompartment zugeordnet werden.

Jedes einzelne Kompartment ist gekennzeichnet durch ein Volumen und ein effektives Tidalvolumen, wobei die Summe der Volumina aller Kompartments die FRC und die Summe der effektiven Tidalvolumina aller Kompartments das effektive Tidalvolumen der Lunge für den betrachteten Auswasch ergibt (Parallelschaltung). Die Berechnung der Tidalvolumina macht vor allem unter regelmäßiger Spontanatmung bzw. mandatorischer maschineller Beatmung Sinn, da in diesen Fällen die Tidalvolumina über den Auswasch nahezu konstant bleiben.

Für eine Lunge mit zwei Kompartments ergibt sich beispielsweise folgender Zeitverlauf der endexspiratorischen Konzentrationen, wobei der Laufindex i die Zahl der Atemzüge seit dem Beginn des Auswaschs bezeichnet, c(0) ist die bekannte Ausgangskonzentration in der Lunge und $\lambda_1$ und $\lambda_2$ sind die zu bestimmenden Zerfallsraten der beiden Kompartments:

$$c(i) = c(0) \cdot e^{-\lambda_1 \cdot i} + c(0) \cdot e^{-\lambda_2 \cdot i}$$

Dekadisches Logarithmieren der Konzentration und Normierung auf die Anfangskonzentration ergibt:

$$\log_{10}\left(c(i)/c(0)\right) = \log_{10}\left(e^{-\lambda_1 \cdot i} + e^{-\lambda_2 \cdot i}\right) = -\lambda_1 \cdot i \cdot \left(\log_{10} e\right) + \log_{10}\left(1 + e^{(\lambda_1 - \lambda_2)\cdot i}\right)$$

Nimmt man ohne Beschränkung der Allgemeinheit an, dass $\lambda_1 > \lambda_2$, also dass $\lambda_1$ die dominierende Zerfallsrate darstellt (schnellster Zerfall), so kann man die erste lineare Komponente extrahieren (erster Summand auf der rechten Seite in obiger Gleichung). Da der Term im Exponenten des zweiten Summanden positiv ist und mit steigendem i wächst, kann man für große i (gegen Ende des Auswaschs) die 1 im Logarithmus vernachlässigen. Für große i ergibt sich näherungsweise:

$$\log_{10}\left(c(i)/c(0)\right) \approx -\lambda_1 \cdot i \cdot \left(\log_{10} e\right) + \left(\lambda_1 - \lambda_2\right)\cdot i \cdot \left(\log_{10} e\right) = -\lambda_2 \cdot i \cdot \left(\log_{10} e\right)$$

Für das Zwei-Kompartment-Modell sind die Unbekannten damit bestimmt. Im Fall von mehr als zwei Kompartments muss man $\lambda_2$ durch die Summe aller Zerfallsraten mit Ausnahme von $\lambda_1$ ersetzen. Die Analyse ergibt dann für große i (Ende des Auswaschs) die kleinste Zerfallsrate, diese kann durch Subtraktion in der Ausgangsgleichung eliminiert werden. Anschliessend bestimmt man in der semilogarithmischen Darstellung die zweitkleinste Zerfallsrate usw. Je größer der Quotient aus größter zu kleinster Zerfallsrate ist, desto größer ist die Inhomogenität der Lunge.

Wenn man das Totvolumen vorher abgezogen hat, erhält man für die Zerfallsraten und die Volumina der Kompartments folgende Beziehungen:

$$\lambda_j = V_{Tj} / V_j$$

$$FRC = \sum_j V_j$$

$$V_T = \sum_j V_{Tj}$$

Hierbei bezeichnet $VT_j$ das effektive Tidalvolumen für das j-te Kompartment, $Vj$ das Volumen des j-ten Kompartments. Für das Zweikompartmentmodell ergeben sich daraus folgende einfache Beziehungen:

$$V_{TI} = \frac{V_T - \lambda_2 \cdot FRC}{1 - \lambda_2 / \lambda_1} \qquad V_I = V_{TI} / \lambda_1$$

$$V_{T2} = \frac{\lambda_I \cdot FRC - V_T}{\lambda_I / \lambda_2 - 1} \qquad V_2 = V_{T2} / \lambda_2$$

Darüber hinaus ergibt sich durch Mengenbestimmung des ausgewaschenen Tracergases über die bekannte Inhalationszusammensetzung eine Möglichkeit zur Berechnung der in die Lunge gelangten Menge an medizinisch wirksamen Bestandteilen, wodurch eine exaktere Dosierung von Inhalationsmedikamenten als bisher möglich wird. Eine weitere Verwendungsmöglichkeit besteht bei abwechselnder oder simultaner Inhalation von "R 134a" oder "R 227", um die unterschiedliche Blutlöslichkeit beider Fluorkohlenwasserstoffe für die Lungenfunktionsdiagnostik zu verwenden.

Die Blutlöslichkeit von Gasen kann beschrieben werden durch den Blood-Gas-Partitionskoeffizienten L, der das Verhältnis der Volumenanteile zwischen in Blut gelöstem und in der Gasphase befindlichem Gas beschreibt. Voraussetzung hierfür ist das Vorliegen eines Gleichgewichts mit identischen Partialdrücken des Gases in der im Blut gelösten und in der Gasphase. Für die Lungenfunktionsdiagnostik und insbesondere die Bestimmung der FRC ist eine möglichst geringe Blutlöslichkeit der Tracergase erwünscht, da man schliesslich das Lungenvolumen und nicht die Blutmenge im Körper bestimmen möchte. Durch Verwendung mindestens zweier Gase mit unterschiedlichen Blutlöslichkeiten lässt sich die FRC-Messung hinsichtlich der Blutlöslichkeit korrigieren. Im folgenden bezeichnen die Indices 1 und 2 die beiden Gase, die z.B. für R 134a und R 227 stehen können.

Nimmt man an, dass für die FRC-Messung nicht nur die Konzentration in der Lunge im Gleichgewicht ist, sondern dass auch die Blutkonzentration ihren Gleichgewichtswert erreicht hat, so ergibt eine FRC-Bestimmung abhängig von der Blutlöslichkeit unterschiedliche Werte:

$$FRC_j = FRC + V_{Blut} \cdot L_j$$

Die mit dem Gas j gemessene $FRC_j$ wird im Vergleich zum wahren Wert (hier mit FRC bezeichnet) um das Produkt aus Blutvolumen im Körper und Blood-Gas-Partitionskoeffizienten L überschätzt. Unter Kenntnis des Blutvolumens kann bereits mit einem Gas bekannter Löslichkeit eine Korrektur der FRC-Bestimmung durchgeführt werden. Mit Hilfe eines zweiten Gases kommt man ohne eine Kenntnis des Blutvolumens aus:

Im Folgenden kann ohne Beschränkung der Allgemeinheit davon ausgegangen werden, dass $L_1 > L_2$, so dass die mit Gas 1 (zum Beispiel R 134a) gemessene FRC größer ausfällt als die mit Gas 2 (zum Beispiel R 227) bestimmte. Aus der Differenz der FRC-Werte lässt sich das Blutvolumen bestimmen:

$$V_{Blut} = \frac{FRC_1 - FRC_2}{L_1 - L_2}$$

Dies ermöglicht die Korrektur der näher am wahren Wert liegenden FRC-Messung mit dem Gas 2:

$$FRC = FRC_2 - \frac{FRC_1 - FRC_2}{L_1 / L_2 - 1}$$

Im Fall von R 134a und R 227 liegt der Koeffizient $L_1/L_2$ etwa bei 4, so dass die gemessene FRC-Differenz durch 3 geteilt wird.

Insbesondere kann neben der FRC-Bestimmung eine Abschätzung des "Pulmonary Blood Flow" durchgeführt oder die FRC-Bestimmung bezüglich der im Blut gelösten Tracergasmenge korrigiert werden.

Die Unteransprüche geben bevorzugte Verwendungen des 1, 1, 1, 2 - Tetrafluorethans an, wobei die genannte Inhalationszusammensetzung speziell eine sich unter Druck befindende flüssige Zusammensetzung mit einem darin gelösten Medikament für die Asthmatherapie oder für die Therapie anderer Lungenerkrankungen ist und die Dosierung mittels eines Spraydosierers, MDI (= Medical Dose Inhaler) bei der Beatmung erfolgt. Speziell erfolgt die Anwendung so, dass bei Annahme einer durch den Patienten aufgenommenen Menge von etwa 10 bis 30 % der pro Dosierstoß dosierten Gesamtgasmenge die Konzentration von 1,1,1,2 - Tetrafluorethan allein oder in Kombination mit 1,1,1,2,3,3,3, - Heptafluorpropan maximal etwa 1 bis 5 Vol. %, speziell 0,1 bis 1 Vol. % des nach der Inhalation sich in der Lunge befindenden Gasgemischs beträgt.

**Patentansprüche**

1. Verwendung von 1,1,1,2-Tetrafluorethan als Spurengas zur Lungenfunktionsmessung bei der Beatmung mittels eines Anästhesie- oder Beatmungsgerätes.

2. Verwendung von 1,1,1,2-Tetrafluorethan nach Anspruch 1 für die FRC-Bestimmung, insbesondere durch Auswaschung.

3. Verwendung von 1,1,1,2-Tetrafluorethan nach Anspruch 1 oder 2, **dadurch gekennzeichnet, dass** das 1,1,1,2-Tetrafluorethan Bestandteil einer Inhalationszusammensetzung ist, speziell mit mindestens einem Medikaments

4. Verwendung von 1,1,1,2-Tetrafluorethan nach einem der vorhergehenden Ansprüche, **dadurch gekennzeichnet, dass** als weiteres Spurengas 1,1,1,2,3,3,3-Heptafluorpropan verwendet wird, insbesondere in einem Mengenverhältnis der beiden Spurengase 1,1,1,2,3,3,3- Heptafluorpropan: 1,1,1,2-Tetrafluorethan von maximal 1 : 2 bis 1 : 3.

5. Verwendung von 1,1,1,2-Tetrafluorethan nach einem der vorhergehenden Ansprüche, **dadurch gekennzeichnet, dass** die Konzentration des Spurengases 1,1,1,2-Tetrafluorethan allein oder in Kombination mit 1,1,1,2,3,3,3-Heptafluorpropan bei der Beatmung maximal etwa 1 bis 5 Vol. %, insbesondere 0,1 bis 1 Vol. % des sich in der Lunge befindenden, eingeatmeten Atemgasvolumens beträgt.

6. Verwendung von 1,1,1,2-Tetrafluorethan nach einem der vorhergehenden Ansprüche, **dadurch gekennzeichnet, dass** dessen Dosierung für die Lungenfunktionsmessung oder die FRC-Bestimmung kontinuierlich oder quasikontinuierlich pro Atemzug mittels eines Spraydosierers während der Beatmung erfolgt.

**Claims**

1. The usage of 1,1,2,2-tetrafluoroethane as a trace gas for lung function evaluation during ventilation using an anaesthesia device or respirator.

2. The usage of 1,1,1,2-tetrafluoroethane according to claim 1 for determination of functional residual capacity (FRC), particularly by washout.

3. The usage of 1,1,1,2-tetrafluoroethane according to claim 1 or 2, **characterized in** said 1,1,1,2-tetrafluoroethane being part of a composition for inhalation, especially in combination with at least one medication.

4. The usage of 1,1,1,2-tetrafluoroethane according to one of the previous claims, **characterized in that** 1,1,1,2,3,3,3-heptafluoropropane is used as an additional trace gas, especially in a proportion of both trace gases 1,1,1,2,3,3,3-heptafluoropropane : 1,1,1,2-tetrafluoroethane of 1 : 2 to 1 : 3 at the maximum.

5. The usage of 1,1,1,2-tetrafluoroethane according to one of the previous claims, **characterized in that** the concentration of the trace gas 1,1,1,2-tetrafluoroethane as such or in combination with 1,1,1,2,3,3,3-heptafluoropropane during ventilation is about 1 to 5 % by volume at the maximum, more preferably 0.1 to 1 % by volume at the maximum, based on the inhaled respiratory gas volume contained in the lung.

6. The usage of 1,1,1,2-tetrafluoroethane according to one of the previous claims, **characterized in that** for lung function evaluation or FRC determination, the dosing thereof during ventilation is continuously or essentially continuously provided by use of a spray dosing unit.

**Revendications**

1. Utilisation de 1,1,1,2-tétrafluoro-éthane en tant que gaz de traçage pour l'évaluation de la fonction pulmonaire lors d'une ventilation effectuée au moyen d'un appareil d'anesthésie ou de ventilation.

2. Utilisation de 1,1,1,2-tétrafluoro-éthane, conforme à la revendication 1, pour la détermination de la capacité résiduelle fonctionnelle, en particulier par lavage.

3. Utilisation de 1,1,1,2-tétrafluoro-éthane, conforme à la revendication 1 ou 2, **caractérisée en ce que** le 1,1,1,2-tétrafluoro-éthane est un constituant d'une composition pour inhalation, comprenant en particulier au moins un médicament.

4. Utilisation de 1,1,1,2-tétrafluoro-éthane, conforme à l'une des revendications précédentes, **caractérisée en ce qu'**on utilise, comme autre gaz de traçage, du 1,1,1,2,3,3,3-heptafluoro-propane, en particulier en une quantité telle que le rapport des quantités de ces deux gaz de traçage, du 1,1,1,2,3,3,3-heptafluoro-propane au 1,1,1,2-tétrafluoro-éthane, vaille au plus de 1/2 à 1/3.

5. Utilisation de 1,1,1,2-tétrafluoro-éthane, conforme à l'une des revendications précédentes, **caractérisée en ce que** la concentration de gaz de traçage, c'est-à-dire du 1,1,1,2-tétrafluoro-éthane seul ou associé au 1,1,1,2,3,3,3-heptafluoro-propane, lors de la ventilation représente au plus à peu près 1 à 5 % et en particulier 0,1 à 1 % du volume de gaz respiratoire introduit par ventilation et présent dans les poumons.

6. Utilisation de 1,1,1,2-tétrafluoro-éthane, conforme à l'une des revendications précédentes, **caractérisée en ce que**, pour l'évaluation de la fonction pulmonaire ou la détermination de la capacité résiduelle fonctionnelle, on effectue le dosage de ce gaz de manière continue ou quasi continue, expiration après expiration, au moyen d'un inhalateur doseur, pendant la ventilation.

Optische Absorptionsspektren

Anästhesiegase (Enfluran, Isofluran, Sevofluran, Desfluran, Halothan): 500 ppm x m

$N_2O$: 2500 ppm x m, R227: 180 ppm x m, R134a: 100 ppm x m

EP 1 759 634 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10046465 B4
- US 6544191 B2
- US 6254546 B1